# EUROPEAN PATENT APPLICATION

(11) **EP 1 199 083 A1**
(43) Date of publication of application: **24.04.2002**
(21) Application number: 01500245.4
(22) Date of filing: 11.10.2001
(51) Int. Cl.: A61M 25/10, A61M 5/48

(54) **Apparatus for the inflation and deflation of balloon catheters and a method for its use**

(30) Priority: 16.10.2000 ES 200002471
(71) Applicant: Probitas Pharma, S.A., 08005 Barcelona (ES)
(72) Inventor: Ferrer Royo, Roger, 08190 Sant Cugat del Valles(Barcelona) (ES); Martinell Gisper-Sauch, Enric, 08010 Barcelona (ES); Manzano Riera, Jordi, 08009 Barcelona (ES)
(74) Representative: Duran Moya, Luis-Alfonso

(57) **Abstract**

The apparatus is characterized in that it comprises, in a single, transportable body provided with a handle, a support for a motor-driven impelling syringe unit connected to a pressure tube, forming a single-use, replaceable element, driven by means of a motor and helical-screw unit the motor of which is operated by means of a control board which receives information relating to the force of the piston of the motor-driven syringe, its positioning, and also acoustic (or phonetic) instructions from a microphone, and electronic instructions from a touch screen which can display the parameters indicative of the progress of the operation for the correction a lesion of an organic duct (artery, vein, bile duct, urological duct, etc.) as well as receiving instructions from the operator.

## Description

The present invention relates to electromechanical apparatus for the inflation and deflation of balloon catheters, of the type which is programmable and which includes a single-use device for the inflation and deflation of balloon catheters in controlled manner and with complete safety, the invention also relating to the method of implementing the invention.

The application of the invention is the inflation and deflation of balloon catheters of various types, such as those used for PTCA, PTA, the implantation of metal prostheses, valvuloplasty, AAA prosthesis, and the like.

In various pathological conditions characterized by reduction of the normal diameter of various anatomical ducts and cavities (arteries, veins, bile ducts, urological ducts, digestive tract, etc.) these constrictions are conventionally dilated by the introduction of a balloon catheter through them.

The balloon of the balloon catheter, which is introduced percutaneously (by puncture through the skin) or through a natural duct, is deflated in a first stage of the procedure to facilitate its forward movement, until it reaches and extends through the lesion to be dilated.

In a second stage, the balloon is inflated, once or several times, with a liquid (usually a physiological solution with a variable % of radiological contrast medium for the display of the procedure by radioscopy) contained in a syringe or in a manual inflator device with a manometer.

Once the lesion has been dilated, the balloon is deflated and withdrawn from the patient's body.

The types of balloons used depend on the lesion to be treated, with low volume and medium or high pressure in PTCA, PTA, and implantation of metal prosthesis, and with high volume and low pressure in cardiac valvuloplasty, and AAA prosthesis implantation...

The balloons of balloon catheters are normally inflated in conventional manner with standard syringes which are not specific to the procedure, or with manual inflators which incorporate a pressure manometer if they are specifically for inflation and deflation. In the first case, there is no measurement of the pressure exerted manually on the piston. In the second case measurement takes place, by reading of the value provided by the manometer (normally in atmospheres or in p.s.i.).

In any case, the display, by fluoroscopy, of the disappearance of the pathological constriction is the indicator of the success of the procedure. That is to say, it is the action on the piston of the syringe, or of the manual inflator that determines the pressure applied at a particular moment. Once the effectiveness of the dilation has been assessed as positive, the balloon is deflated by the application of a negative pressure to the piston of the syringe or of the manual inflator.

The means and the devices known up to now have various disadvantages of which the main ones are:
- Lack of objective control of the pressure applied with the use of a standard syringe, or imprecise control for the manual inflator with manometer; the application of insufficient pressure may cause partial dilation of the lesion; the application of excessive pressure may cause mechanical damage in the organic structure (walls of vessels, ducts, etc.); on the other hand, there is a physical difficulty in applying high pressures manually; this difficulty is less in a manual inflator, the piston of which is operated by rotation of the rod which links it to the operator's hand;
- Lack of objective control of the dilation time since, with the use either of a syringe or of an inflator, the operator observes the inflation of the balloon purely visually, in order to determine when the lesion being corrected is dilated; the method usually used consists of the application of a predetermined, but arbitrary, approximate time which may last from a few seconds to several minutes;
- Unreliable control of bursting of the balloon, which is one of the possible complications that may arise owing to failure of the material of which the balloon is made or to an excess of pressure thereon; the conventional method does not enable the effects which bursting may have on the tissues surrounding the balloon to be minimized;
- Difficulty in manipulation since the currently known method requires the use of the operator's hands, one for gripping the body of the syringe or manual inflator, and the other for controlling the forward or rearward movement of the piston;
- Unreliability in the recording of the data relating to the progress of the method, such as times and pressures, which conventionally are recorded manually, which represents the possible loss of data, inaccuracy in the recording thereof, etc.

The present invention is intended to overcome the above-mentioned disadvantages by proposing a set of means which comprise apparatus, a sterile device, and a method of implementing the invention, and which can achieve effective objective control of the pressure applied and also of the dilation of the inflatable element, and simplified manipulation, leaving the operator free.

In summary, the apparatus is a small-format electromechanical device which, when installed on or close to the operating table of a catheterization theatre, enables the operator (skilled medical personnel: haemodynamicist cardiologist, vascular radiologist, vascular neuroradiologist, etc.) to achieve the inflation and deflation of balloon catheters in manner which is controlled, precise, effective and safe for the patient. The design of the apparatus, as well as its various functions provide all of the information (visual by means of a screen, phonetic and acoustic by means of a loudspeaker; it also has an outlet for the export of data relating to the procedure) and control devices (touch screen, microphone for spoken commands, and remote control sensitive to the degree of pressure on its buttons) which is necessary for the operator to perform the balloon-catheter inflation and deflation procedure, adapting it to the actual circumstances of the type of lesion to be treated and to the specialist's technical criteria on the basis of his experience and knowledge.

The invention also includes a sterile, single-use device composed basically of a high-pressure syringe and of a flexible tube, also for high pressure, fixed firmly to the syringe or connected thereto by means of a Luer-lock connection. At the other end (the distal end) the tube may have a three-way valve which facilitates purging of the catheter and the syringe.

The device described must be filled with a liquid solution (generally a physiological saline solution and an iodized, radiological contrast solution), eliminating as far as possible the presence of air in the device (purging).

The action (forwards and backwards) of the mechanical element of the inflation apparatus on the piston of the inflator device enables the balloon catheter to be inflated and deflated upon the application of a positive or negative pressure, according to the stage of the method.

The method consists basically of a series of successive steps which are indicated briefly below:
a) Preparing the apparatus (setting in operation and checking condition) and the single-use device (filling with the solution provided for, and purging of the air);
b) Preparing the balloon catheter (purging and inspection of its characteristics, basically, of the maximum pressure permitted);
c) Preparing the remote control, checking its operative condition and correct operation (visual confirmation by means of the screen);
d) Purging the syringe and the pressure tube, eliminating as far as possible any air which they may contain;
e) Loading the single-use syringe in the inflator apparatus; gaining access to the housing by lifting its transparent cover; closing the cover once the syringe is fixed;
f) Selecting the characteristics of the procedure by means of the touch screen (automatic or manual mode, maximum safety pressure, speed or rate of increase of the pressure, measurement units, acoustic warning, graphic display of the dilation, and the like);
g) Connecting the end of the flexible pressure tube of the syringe to the balloon catheter, already inserted in the patient with its balloon extending through the lesion to be dilated;
h) Starting dilation by operating the button for setting the remote control in operation; if the pressure is to be increased uniformly, applying a constant pressure; if the pressure is to be increased non-uniformly, applying a greater or lesser pressure to the button; when pressure ceases to be exerted on the button, the dilation pressure is kept constant and an internal clock of the inflator apparatus starts its count in seconds until the operator operates the deflation button;
i) The procedure may include several manual or automatic inflation and deflation cycles;
j) If the procedure has been satisfactory, disconnecting the pressure tube from the catheter, recording the data obtained by the inflator apparatus and withdrawing the syringe.

In implementing the method, a maximum safety pressure will have to be set.

The invention allows for the method to be performed in several ways, as indicated below.

Manual operative mode: the procedure is controlled entirely by the action of the operator on the remote control; as a safety criterion, the same functions which are programmed for the remote control may be performed by means of the touch screen, that is to say, if the remote control fails, it is possible to operate by means of the screen.

Semi-automatic operative mode: when the dilation pressure, the rate of increase of the pressure, and the dilation time have been programmed, and once the programmed pressure has been reached, the inflator apparatus awaits the command to start the procedure by means of the remote control; this command may be given by means of the touch screen if desired.

From this moment, the method proceeds automatically; however, it is possible to change to manual mode at any moment by pressing the remote-control button.

Automatic operative mode: the inflator apparatus increases and controls the inflation pressure automatically until the lesion is dilated.

For this operative mode, it is possible to program the rate of increase of the pressure (constant or in two stages so that, once the inflator apparatus detects a predetermined increase in the pressure, it applies a second rate of pressure increase, or transfers the inflation to the operator at this point), as well as a period of time for which to maintain the dilation pressure (holding time), as desired.

It is possible to change to manual mode at any moment by pressing the remote-control button.

The inflator can detect the presence of an excessive volume of air in the system formed by the catheter, the pressure tube, and the syringe by interpretation of the variation of the pressure in relation to the variation in volume.

At the appropriate time, it allows the maximum safety pressure, which will not be exceeded by the inflator, to be selected. The selection and programming of this function is protected by a restricted-access key. As a safety measure, if the maximum pressure is reached, the inflator issues an acoustic warning and a visual warning on the screen.

The apparatus permits the selection of a dilation pressure which, once reached, will remain constant until the moment at which the operate decides to modify it. The apparatus has means for issuing an acoustic warning and a visual warning which is displayed on the screen when the dilation pressure has been reached.

In the method for the use of the apparatus, the operator may select a dilation time at the dilation pressure. There are also means for issuing an acoustic warning and a visual warning on the screen once the preset time has elapsed.

It is also possible to select the type of syringe to be used, as appropriate for the operative case.

The apparatus also permits programming of a ramp for the increase of the pressure or of the volume, or for the movement or travel of the piston of the syringe, which will determine the time elapsing between the start of inflation, or zero time, to the moment at which the selected dilation pressure is reached.

It is also possible to select the method of display of the basic data which may be displayed on the screen in digital form with windows with alphanumeric digits, or in analog form by a simulation of a pressure manometer resembling that of a conventional inflator, permitting rapid and simple display of the inflation in a form resembling the conventional form.

If graphic display is selected, it is possible to proceed as shown in Figures 3, 4 and 5.

When this option is selected, a graph of the dilation curve obtained by comparing the variation of the pressure (Y axis) with the variation in the volume injected (X axis) is presented on the screen.

A characterization of the dilation is thus obtained, from which it is possible to derive clinical interpretations such as the plasticity of the lesion (curve with a greater or lesser slope; Figure 3), the pressure at which the lesion dilates (Figure 4), or the effectiveness of the dilation (comparison of two or more successive dilations in the same lesion; Figure 5).

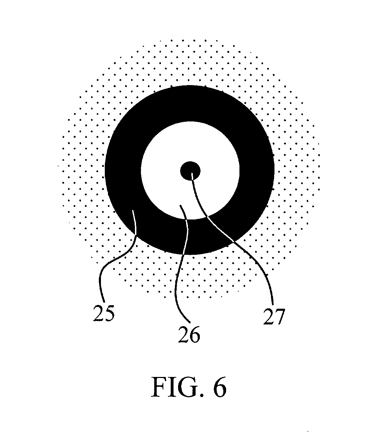

The operator can select the parameters to compare and also the preferred units and scales. If axial representation of the dilation is selected, as shown in Figure 6, the dilation procedure is reproduced on the screen by the display of concentric rings. As shown in Figure 6, the outer annular space 25 represents the cavity or cross-section corresponding to the natural or healthy duct. The intermediate annular area 26 represents the cavity or cross-section of the stenosed or constricted duct, and the inner, smaller circle 27 represents the membrane of the catheter balloon.

Reference values: in order for the inflator faithfully to reproduce the actual diameters and the relationship between them, reference values obtained by measurements made in images originating from various diagnostic imaging techniques (angiography, echography, TAC, magnetic resonance, etc.) or by inflation of the balloon in the distal healthy section adjacent the lesion, must be programmed (entered).

Visual codes: the various circles, as well as the areas which they delimit may be represented with the use of different colours, textures, and hatching (to be selected by the user), which facilitate its visual interpretation.

The greater or lesser plasticity of the lesion and its greater or lesser eccentricity are represented on the screen with the use of different characteristic colours, and/or alphanumeric messages.

For a better understanding thereof, some drawings of the apparatus and the method of the present invention are appended by way of non-limiting example.

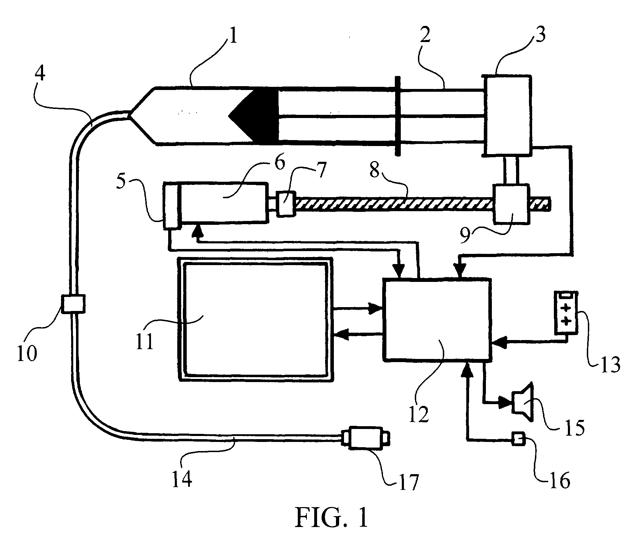

Figure 1 shows, in a simplified manner, the set of elements which make up the apparatus of the present invention, and which enable the method of the invention to be implemented.

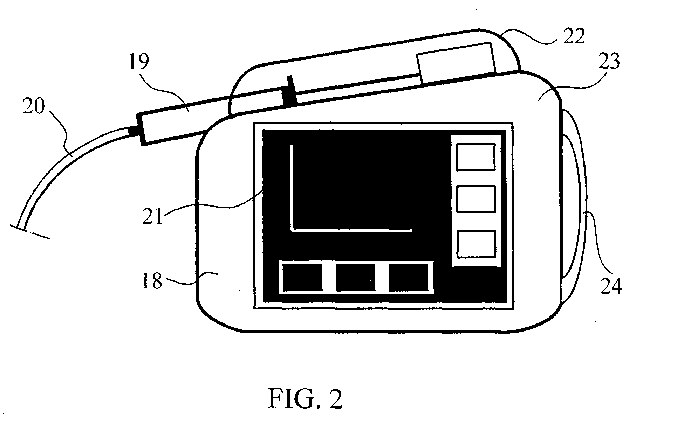

Figure 2 is a simplified view of the apparatus of the present invention.

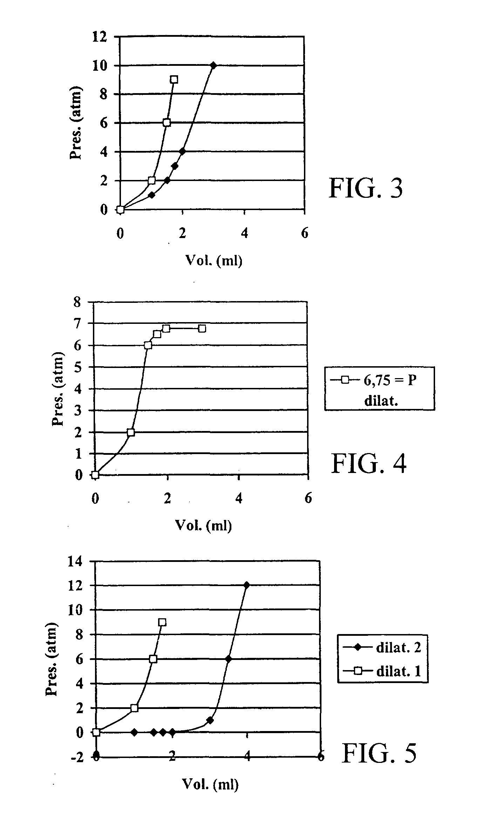

Figure 3 shows a characterization of the dilation for deriving an interpretation of the plasticity, distinguishing between hard and soft lesions, which are represented by a light square and a dark diamond, respectively.

Figure 4 shows a graphical characterization of the dilation with an indication of the pressure at which dilation takes place.

Figure 5 is a graphical representation of the effectiveness of the dilation by comparison of two or more successive dilations of the same lesion.

Figure 6 is a schematic representation of the dilation in cross-section.

According to the drawings and, in particular, according to the schematic view of Figure 1, the apparatus of the invention comprises a disposable unit which is composed of a syringe 1 and a high-pressure tube 4 and which supplies, by means of a valve 10, a catheter 14 with an inflatable balloon device 17, which is disposed at the distal end of the pressure tube. The apparatus is purged with a solution of variable concentration, preferably saline solution with a radiological contrast medium. A supply, controlled by an electronic board 12, generates the voltage necessary to rotate a direct-current motor 6 which drives a screw 8 by means of a coupling 7. The rotation of the screw is translated into a linear movement of a body 3 housing a piston 2 of the syringe 1, by means of a nut 9 coupled with the helical screw 8. The piston 2 is housed in the receiving or housing body 3 in a manner such that both move together. The body 3 housing the piston comprises a load cell, not shown, which converts the force exerted on the piston 2 into an electrical signal which is processed by the control board 12 in a manner such that, if the diameter of the piston 2 and the force exerted thereon are known, it is possible to obtain indirectly the pressure of the fluid inside the syringe which, owing to the very low compressibility of the liquid, will be equal to that exerted by the balloon 17 on the lesion. The electronic control board 12 also receives information relating to the absolute position of the piston 2 of the syringe 1, by means of an optical encoder 5 fixed to the shaft of the direct-current motor 6.

Exchange of information with the operator takes place by means of a touch screen 11, a remote control 13, provided, for example, with three buttons, a loudspeaker 15, and a microphone 16. The operator generates inflation, deflation and emergency-stop commands by means of three buttons of the remote control 13. It is also possible to select predetermined non-critical options by voice recognition which the electronic control board 12 performs from the signal picked up by the microphone 16. All of the functions of the inflator can also be programmed by means of the touch screen 11, the information output by the inflator being displayed on the touch screen 11 or represented by means of a voice synthesizer of the electronic control board 12, with loudspeaker output 15.

The apparatus adopts the structure shown in Figure 2 which shows an enveloping body 18 to which a syringe unit 19 and a pressure tube 20 which supplies the balloon catheter, not shown, are connected. A touch screen 21 provides colour graphical information relating to the characteristics of the method such as the pressure applied in relation to the volume of solution injected, and the time elapsed, and the like.

The screen provides numerical information relating to the working values (instantaneous pressure, maximum pressure programmed, time elapsed from the start of the inflation of the balloon, time elapsed from the moment at which the desired dilation pressure was reached, etc.) to the state of the apparatus (initialization, ready for loading of the disposable device, ready for inflation, procedure completed, etc.) and to incidents connected with the safety of the procedure which may occur (bursting of the balloon, operating or programming errors, lack of energy supply, values outside the limits established by the operator, etc.).

The operator can program and control the functions provided for which, basically, are the selection of the maximum safety pressure, of the type of method (manual or automatic), of the start and conclusion of the inflation, of the display of the graphs of the procedure, of the units of pressure (atmospheres, mmHg, p.s.i.), of time (seconds, minutes), and of volume (ml, c.c.), of the summary of the procedure, of error information and output and, in general of all of the processes provided for in the operation of the apparatus.

The luminosity, colour and response characteristics of the screen provide the operator with clear, easily-read information, at any moment and without dead display angles.

The apparatus has a housing for the single-use device for which, in the upper portion of the inflator apparatus and protected by a transparent cover 22, it has a recess for receiving the syringe of the single-use device. Once the syringe is filled with the solution provided for (physiological solution and radiological contrast medium) and any air present has been eliminated (purged), the syringe is placed manually in the housing in a manner such that the proximal end of the piston fits into the receptacle (now in the loading position) of the element for moving the inflator apparatus forwards and backwards. The apparatus is operative only if it is possible to close the transparent cover and the closure of the latter is possible only if the syringe is arranged appropriately in the housing.

The outer casing 23 of the apparatus has a design depending upon the shape of the screen and upon the protection of the set of the elements, with a handle 24 which enables the operator to orient the apparatus according to his preferences. Control members such as the on/off power switch, the connection for the network cable, the connection for the remote control, and the data output are situated in the rear cover.

The manually-operable control enables inflation to be started and maintained (either with automatic operation or with manual operation) and enables the rate of increase of the inflation pressure to be selected by pressure on the button provided. The control incorporates an emergency button which enables the positive pressure exerted by the syringe to be eliminated instantaneously if necessary.

The control has a cable of sufficient length to connect it to the inflation apparatus.

If the acoustic warning device of the apparatus, which is not shown in Figure 2, is activated by the operator by means of the touch screen, after a certain period time, for example, a few seconds, it will notify the elapsed dilation time and the pressure reached. Information relating to the operative state, and the like, may also be provided.

The inflator apparatus has a support system which is preferably fixed to the catheterization table, although the design of other supports which enable it to be fixed to the ceiling or to a wall of the catheterization theatre are provided for. The support system has, at its connection with the inflation apparatus, an articulation which enables the apparatus to be oriented in accordance with the operator's criteria.

The sterile, single-use device for the inflation and deflation of the balloon catheter comprises a high-pressure syringe (nominally permitting 25 atm.) with a nominal volume of 20 ml, although the use of other syringes with different volumes (30, 40, or 50 ml) is provided for. The material of which the body of the syringe is manufactured is transparent, enabling the presence of any air-bubbles inside it to be detected visually. The syringe may have, at its output connection, a flexible, high-pressure tube connected firmly thereto (sealed with adhesives).

The syringe indicated 1 in Figure 1 is connected, by means of the high-pressure flexible tube 4, which may have a connection to the catheter or two connections, one for the catheter and the other for the syringe. The tube is transparent and may have, at its distal end, a three-way valve for the purging of the catheter and of the syringe.

The apparatus of the present invention is characterized in that it has means for the interpretation of the variation of the pressure in relation to the variation of the volume in order to detect air in the system, to obtain the change in the lesion, the effectiveness of the dilation, the characterization of the lesion, and detection of the moment at which the lesion yields under the dilation pressure.

In the actual operation of the apparatus, it will perform the following operations:

### 1. Detection of the presence of air.

The inflator can detect the presence of an excessive volume of air in the system formed by the catheter, the pressure tube, and the syringe, by interpretation of the variation of the pressure in relation to the variation of the volume. Leaks in the system can also be detected.

### 2. Selection of the maximum safety pressure.

The operator can select a maximum safety pressure which may not be exceeded by the inflator in any circumstances. The selection and programming of this function are protected by a restricted access key.

If the maximum safety pressure is reached, the inflator emits an acoustic warning and displays a warning on the screen.

### 3. Selection of the dilation pressure.

The operator can select a dilation pressure which, once it has been reached, remains constant until the moment at which the operator decides to modify it (by increasing or decreasing it).

When the dilation pressure has been reached, the inflator emits an acoustic warning and displays a warning on the screen.

### 4. Selection of the holding time.

The operator can select a time in minutes and/or seconds for dilation at the dilation pressure. When this time has elapsed, the inflator emits an acoustic warning and displays a warning on the screen.

### 5. Selection of the type of syringe, according to its capacity.

The operator can select the type of syringe to be used from the various models provided for.

### 6. Selection of the rate of increase of the pressure.

The operator can program a ramp for the increase of the pressure or of the volume (or of the movement or of the travel of the syringe piston) which will determine the time elapsing between the start of inflation (time 0) and the reaching of the selected dilation pressure.

### 7. Selection of the method of display of the basic data.

The operator can select to display the basic data of the method on the screen in two possible ways:
* Digital: the data is displayed in windows with alphanumeric digits.
* Analog: the data is represented in a simulation of a pressure manometer resembling to those of conventional inflators. This option enables the inflation procedure to be displayed in a manner similar to the conventional method.

## Claims

1. Apparatus for the inflation and deflation of balloon catheters and a method for its use, the apparatus being of the type comprising a pump for a fluid for the inflation of the balloon, the pump being connected to the catheter for supplying the balloon, with motor-driven means for actuating the pump and a control device, **characterized in that** it comprises, in a single transportable body, provided with a handle, a support for a motor-driven impelling syringe unit connected to a catheter, forming a single-use, replaceable element, driven by means of a motor and helical screw unit the motor of which is operated by means of a control board which receives information relating to the force of the piston of the motor-driven syringe, its positioning, and also phonetic instructions from a microphone and electronic instructions from a touch screen which can display the parameters indicative of the progress of the operation for the correction of a lesion of an organic duct as well as receiving instructions from the operator.

2. Apparatus according to Claim 1, **characterized in that** the piston of the single-use syringe element for impelling the inflation fluid is coupled, at its free end, with a receiving block connected firmly to a female-threaded element which can be driven by the screw driven by the motor.

3. Apparatus according to the preceding claims, **characterized in that** the support for receiving the free end of the piston is coupled to a force detector, connected to the control board, for detecting the force exerted by the piston for impelling the fluid towards the catheter.

4. Apparatus according to Claim 1, **characterized in that** the drive motor is coupled to an optical encoder for detecting the absolute position of the piston which is conditional upon the rotation of the motor and which is transmitted to the control board.

5. Apparatus according to Claim 1, **characterized in that** it comprises means for the exchange of information with the operator by means of the touch screen, a control with manually-operable buttons, a microphone for transmitting non-critical verbal commands, coupled to a voice analyzer, and a voice synthesizer connected to a loudspeaker which can transmit verbal indications to the operator.

6. Apparatus according to Claim 5, **characterized in that** the control with manually-operable buttons comprises three buttons (which are sensitive to the degree of pressure) for bringing about the inflation and deflation of the catheter and emergency stoppage of the apparatus, respectively.

7. Apparatus according to Claim 1, **characterized in that** the single-use pump unit is composed of a high-pressure syringe and a flexible tube fixed firmly thereto.

8. Apparatus according to Claim 1, **characterized in that** the single-use pump unit is composed of a high-pressure syringe and a flexible tube fixed thereto by means of a Luer-lock connection.

9. Apparatus according to Claim 1, **characterized in that** the high-pressure tube for the connection of the high-pressure syringe to the catheter has, at its distal end, a three-way valve for facilitating purging of the catheter and of the syringe.

10. Apparatus according to Claim 1, **characterized in that** the single-use syringe unit is closed by a substantially transparent cover.

11. Apparatus according to Claim 1, **characterized by** the provision of a manually-operable remote control which enables the members for the control and emergency stoppage of the apparatus to be operated remotely.

12. Apparatus according to Claim 1, **characterized in that** an internal clock is provided for counting the time from the start of the inflation operation, the time being displayed on the touch screen.

13. Apparatus according to Claim 1, **characterized in that** it comprises means for the interpretation of the variation in pressure in relation to the variation in volume in order to display it on the touch screen, enabling the presence of air in the system to be detected.

14. Apparatus according to Claim 1, **characterized by** the provision of means for the interpretation of the variation of the pressure in relation to the variation in volume in order to detect air in the system, to obtain the change in the lesion, the effectiveness of the dilation, characterization of the lesion, and detection of the moment at which the lesion yields under the dilation pressure.

15. Apparatus according to Claim 1, **characterized in that** it comprises means for the selection of the maximum safety pressure, the means being protected by a restricted-access key, and being connected to acoustic warning means connected to the loudspeaker and optical warning devices displayed on the screen for warning that the maximum pressure has been reached.

16. Apparatus according to Claim 1, **characterized in that** it comprises means for the selection of the dilation pressure and for keeping the dilation pressure at a constant value until it is modified by the operator.

17. Apparatus according to Claim 1, **characterized in that** it comprises means for the selection of the period of dilation at the dilation pressure.

18. Apparatus according to Claim 1, **characterized in that** it comprises means for the programming of a ramp for the increase of the pressure, of the volume, or of the movement or travel of the piston, which will determine the time elapsing between the start of the inflation and the moment at which the selected dilation pressure is reached.

19. Apparatus according to Claim 1, **characterized in that** it comprises means for displaying the basic data on a screen in digital form with windows with alphanumeric digits.

20. Apparatus according to Claim 1, **characterized in that** it comprises means for the analog representation of the basic data by a simulation of a pressure manometer similar to a conventional inflator on screen for rapid display.

21. An angiography method using the apparatus according to Claims 1 to 19, **characterized in that** the following steps are performed in succession and in coordinated manner:
a) setting in operation and checking of the condition of the apparatus and preparation of the single-use device;
b) purging and inspection of the balloon catheter;
c) preparing the remote control by covering it with a sterility cover and checking its operative condition by means of the visual screen;
d) purging the syringe and pressure tube, eliminating the air;
e) loading the single-use syringe in the inflator apparatus, lifting the transparent cover for access to the housing and subsequently closing the cover;
f) selecting, by means of the touch screen, the characteristics of the method, including manual or automatic mode, maximum safety pressure, rate of increase of pressure, measurement units, acoustic warning, graphic presentation of the dilation, and the like;
g) connecting the end of the flexible pressure tube to the balloon catheter, already inserted in the patient with its balloon extending through the lesion to be dilated;
h) starting dilation by operating the button for setting the remote control in operation;
i) for the manual method, applying a constant pressure, or a greater or lesser pressure to the button, if the pressure is variable;
j) for the manual method, keeping the dilation pressure constant by when the remote-control button ceases to be pressed;
k) starting the count of the internal clock of the apparatus after releasing the remote control button in order to count the time in seconds;
l) deflation of the balloon and repetition of the procedure described above if necessary and until completion of the operation.
